(19) Europäisches Patentamt
European Patent Office
Office européen des brevets

(11) Veröffentlichungsnummer : **0 282 495 B1**

# (12) EUROPÄISCHE PATENTSCHRIFT

(45) Veröffentlichungstag der Patentschrift : **19.06.91 Patentblatt 91/25**

(51) Int. Cl.$^5$ : **A61K 47/00**, A61K 31/415, A61K 35/78, // (A61K35/78, 31:415)

(21) Anmeldenummer : **87904024.4**

(22) Anmeldetag : **24.06.87**

(86) Internationale Anmeldenummer : **PCT/DE87/00284**

(87) Internationale Veröffentlichungsnummer : **WO 88/00473 28.01.88 Gazette 88/03**

## (54) XYLOMETAZOLINHALTIGE PHARMAZEUTISCHE PRÄPARATE.

(30) Priorität : **18.07.86 DE 3624624**

(43) Veröffentlichungstag der Anmeldung : **21.09.88 Patentblatt 88/38**

(45) Bekanntmachung des Hinweises auf die Patenterteilung : **19.06.91 Patentblatt 91/25**

(84) Benannte Vertragsstaaten : **AT BE CH DE FR GB IT LI LU NL SE**

(56) Entgegenhaltungen :
**EP-A- 0 143 305**
**ROTE LISTE, 1986, Editio Cantor, (Aulendorf, Württ.,DE), siehe Nr. 71 040: "Pertix-Hommel Nasentropfen"**

(73) Patentinhaber : **ASCHE AKTIENGESELLSCHAFT**
**Fischersallee 49-59 Postfach 50 01 32**
**W-2000 Hamburg 50 (DE)**

(72) Erfinder : **LOEBEL, Klaus-Dieter**
**Heinrich-Heine-Weg 41**
**W-2050 Hamburg 80 (DE)**

EP 0 282 495 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Xylometazolin = 2-[4-(1,1-Dimethylethyl)-2,6-dimethylphenyl)-methyl]-4,5-dihydro-1H-imidazol ist bekanntlich eine α-spasmomimetisch und vasokonstriktorisch wirksame Verbindung, die unter anderem in Rhinologica Anwendung findet (Anal. Profiles Drug Subst. 14, 1985, 135-156 ; "Rote Liste 1986" ; Editio Cantor Verlag, D-7960 Aulendorf Nr. 71038, Nr. 71033 und Nr. 71039).

Da Xylometazolin in neutralem Milieu nur eine relativ geringe Stabilität besitzt (Acta Pol. Pharm. 38, 1981, 95-98-ref. C.A. 95, 1981, 209 471k) wird es in Rhinologica als Hydrochlorid in wässriger Losung angewendet. Dies hat die Nachteile, daß diese Präparate zur Erzielung einer ausreichenden Lagerfähigkeit Konservierungsstoffe benotigen und daß sie durch Austrocknen der Schleimhaute nicht unerhebliche Beschwerden verursachen.

Es wurde nun gefunden, daß man ohne Zuhilfenahme von Konservierungsstoffen überraschenderweise stabile, lagerfähige Xylometazolin-Base enthaltende pharmazeutische Präparate herstellen kann. Diese Präparate sind dadurch gekennzeichnet, daß sie 0,01 bis 0,5 Gewichtsprozent Xylometazolin-Base und 0,00 bis 3,00 Gewichtsprozent ätherisches Öl in einem Fettsäuretriglycerid der allgemeinen Formel

$$\begin{array}{l} CH_3(CH_2)_xCOO-CH_2 \\ CH_3(CH_2)_yCOO-CH \\ CH_3(CH_2)_zCOO-CH_2 \end{array}$$

worin x, y und z jeweils eine Zahl von 5 bis 8 darstellt, gelost enthalten.

Diese Fettsäuretriglyceride können solche sein, in denen x, y und z jeweils die gleiche Bedeutung besitzen, wie zum Beispiel das Önanthsauretriglycerid, das Caprylsauretriglycerid oder das Caprynsäuretriglycerid ; es ist aber ebensogut möglich Fettsäuretriglyceride der obigen Formel zu verwenden, in denen x, y und z unterschiedliche Bedeutung besitzen, wie dies in den meisten derartigen Neutralölen der Fall ist. Derartige Fettsäuretriglyceride sind beispielsweise die handelsüblich erhältlichen Neutralöle wie das Softenol® 3107 (Hersteller: Dynamit Nobel), das Mygliol® 810/812 (Hersteller : Dynamit Nobel) das Myritol® 318 (Hersteller : Henkel) und insbesondere das Labrafac® lipo (Hersteller : Gattefosse)

Diese öligen Xylometazolin-Lösungen können erfindungsgemäß zusatzlich noch 1 bis 3 ätherische Die enthalten.

Solche Öle sind beispielsweise :

| | Konzentration |
|---|---|
| Eucalyptusöl | 0.01 - 2 % |
| Menthol | 0.01 - 1 % |
| Campher | 0.01 - 1 % |
| Latschenkieferöl | 0.01 - 2 % |
| Fichtennadelöl | 0.01 - 2 % |
| Pfefferminzöl | 0.01 - 3 % |
| Thymol | 0.01 - 0.5 % |
| Salbeiöl | 0.01 - 2 % |
| Kamillenöl | 0.01 - 3 % |

| | Konzentration |
|---|---|
| Fenchelöl | 0,05 - 1 % |
| Cedernblattöl | 0,05 - 1 % |
| Anisöl | 0,01 - 1 % |
| Citronenöl | 0,01 - 1 % |
| Terpentinöl | 0,01 - 1 % |
| Muskatnußöl | 0,01 - 1 % |

Die Konzentrationen sind jeweils in Gewichtsprozent bezogen auf die Gesamtmischung angegeben.

Die Herstellung der erfindungsgemäßen Xylometazolinhaltigen pharmazeutischen Präparate erfolgt, indem man die Komponenten in konventioneller Weise mischt, sterilfiltriert und in der für Xylometazinhaltige Pharmazeutica üblichen Dosis (0,5 ml bis 50 ml) in geeignete Behälter (wie zum Beispiel Flaschen, Dosen, Pipettenflaschen, Spraydosen, Pumpdosiersprüher. Dosiersprayflaschen, Einzeldosierpipetten) abfüllt.

Die anzuwendende Dosis der erfindungsgemäßen Xylometazolinhaltigen Präparate ist die gleiche, wie bei konventionellen Präparaten, die diesen Wirkstoff als Hydrochlorid enthalten.

Die nachfolgenden Ausführungsbeispiele dienen zur näheren Erläuterung der Erfindung.

Beispiel 1

0,5 g Xylometazolin werden bei Raumtemperatur unter Rühren (ca. 300 Umdrehungen pro Minute) in 999,5 g Labrafac® lipo gelöst (Dauer ca. 30 Minuten), sterilfiltriert.

Je 10 ml der so hergestellten Losung wird in eine Pipettenflasche abgefüllt.

Beispiel 2

0,5 g Xylometazolin und 0,5 g Eucalyptusöl werden wie im Beispiel 1 beschriebe in 999 g Labrafac® lipo gelost, sterilfiltriert.

Je 10 ml der Losung wird in eine Dosiersprayflasche abgefullt

Beispiel 3

0,5 g Xylometazolin, 0,5 g Eucalyptusöl und 1,5 g Menthol werden wie im Beispiel 1 beschrieben in 997,5 g Labrafac® lipo gelost, sterilfiltriert.

Je 10 ml der Lösung wird in eine Pipettenflasche abgefüllt

Beispiel 4

1,0 g Xylometazolin und 0,5 g Eucalyptusöl werden in 999 ml Labrafac® lipo gelöst wie in Beispiel 1 beschrieben, sterilfiltriert und zu 10 ml in Dosiersprayflaschen abgefüllt

Beispiel 5

1,0 g Xylometazolin und 0,5 g Eucalyptusöl werden in 988,5 g Labrafac® lipo gelöst, sterilfiltriert und zu 10 ml in Pipettenflaschen abgefüllt.

Beispiel 6

1,0 g Xylometazolin, 0,5 g Eucalyptusöl und 1,5 g Menthol werden in 997 g Labrafac® lipo gelöst, wie in Beispiel 1 beschrieben, sterilfiltriert und zu 10 ml in Pipettenflaschen abgefüllt.

## Ansprüche

1. Xylometazolinhaltige pharmazeutische Präparate, dadurch gekennzeichnet, daß sie 0,001 bis 0,5 Gewichtsprozent Xylometazolin-Base und 0,00 bis 3,00 Gewichtsprozent ätherisches Öl in einem Fettsäuretriglycerid der allgemeinen Formel

$$\begin{array}{l} CH_3-(CH_2)_x-COO-CH_2 \\ \qquad\qquad\qquad\qquad\quad | \\ CH_3-(CH_2)_y-COO-CH \\ \qquad\qquad\qquad\qquad\quad | \\ CH_3-(CH_2)_z-COO-CH_2 \end{array}$$

worin x, y und z jeweils eine Zahl von 5 bis 8 darstellt, gelöst enthalten.

## Revendications

1. Des préparations pharmaceutiques contenant de la Xylométazoline et qui se caractérisent par leur teneur en Xylométazoline-base (0,001% à 0,5% de poids) et en 0% à 3% d'huile éthérée dissolue dans un triglycéride de la formule générale

$$\begin{array}{l} CH_3-(CH_2)_x-COO-CH_2 \\ \qquad\qquad\qquad\qquad\quad | \\ CH_3-(CH_2)_y-COO-CH \\ \qquad\qquad\qquad\qquad\quad | \\ CH_3-(CH_2)_z-COO-CH_2 \end{array}$$

x, y, z représentent respectivement un chiffre entre 5 et 8.

## Claims

1. Pharmaceutical products containing xylometazol ine with the distinguishing characteristic that they comprise 0.001 to 0.5% by weight of xylometazoline base and 0.00 to 3.00% by weight of volatile oil dissolved in triacyl glycerol with the general formula

$$\begin{array}{l} CH_3-(CH_2)_x-COO-CH_2 \\ \qquad\qquad\qquad\qquad\quad | \\ CH_3-(CH_2)_y-COO-CH \\ \qquad\qquad\qquad\qquad\quad | \\ CH_3-(CH_2)_z-COO-CH_2 \end{array}$$

x, y and z standing each for a figure from 5 to 8.